# EUROPEAN PATENT APPLICATION

(11) **EP 4 443 162 A1**
(43) Date of publication of application: **09.10.2024**
(21) Application number: 23382324.4
(22) Date of filing: 03.04.2023
(51) Int. Cl.: G01N 33/574

(54) **GENETIC SIGNATURE FOR PREDICTING THE RESPONSE TO IMMUNOTHERAPIES IN A SUBJECT**

(71) Applicant: Fundación Para La Investigación Biomédica Del Hospital Clínico San Carlos, 28040 Madrid (ES)
(72) Inventor: Ocaña Fernández, Alberto, Madrid (ES); Pérez Segura, Pedro, Madrid (ES)
(74) Representative: Pons

(57) **Abstract**

The present invention relates to methods relating to the prediction of the response of a subject to immunotherapies, preferably immune checkpoint inhibitors, wherein said methods are based on the determination of the expression levels of a set of genes, and subsequent comparison with control values. Furthermore, the invention also relates to a kit comprising means for determining the expression levels of said genes.

## Description

The present invention relates to the prediction of the response of subjects to immunotherapies based on the use of the expression levels of a set of genes. Therefore, the invention could be included in the technical field of immunotherapies and personalized medicine.

### BACKGROUND ART

The immune response to cancer depends on the interaction of the tumor with the host microenvironment. This interaction dictates the immune reaction against cancer and depends on different factors, one of them being the genomic alterations that the tumor itself harbors. Tumors with high genomic instability are those that produce more neoantigens, and therefore prime antigen-presenting cells that subsequently activate effector T-cells. However, oncogenic genomic alterations such as HER2 amplification, or mutations in the EGFR or BRAF gene, among others, induce immune response-modulating phenotypic changes, leading at times to an inhibited immune microenvironment (Kumagai S., Koyama S., Nishikawa H. Antitumour immunity regulated by aberrant ERBB family signalling. Nat Rev Cancer. 2021;21(3):181-197. doi:10.1038/s41568-020-00322-0).

Several types of cells are involved in the host's immune response against cancer. For example, the presence of tumor-infiltrating lymphocytes (TILs) is associated with a better outcome in some types of cancer (Ocaña A., Diez-Gónzález L., Adrover E., Fernández-Aramburo A., Pandiella A., Amir E. Tumor-Infiltrating Lymphocytes in Breast Cancer: Ready for Prime Time? J Clin Oncol. 2015;33(11):1298-1299. doi:10.1200/JCO.2014.59.7286). Similarly, reduction of inhibitory signals, acting with immunotherapies based on immune checkpoint inhibitors, such as antibodies targeting PD-1 (Programmed Cell Death Protein 1), its ligand PDL-1, or CTLA-4 (Cytotoxic T-Lymphocyte Antigen 4), has been shown to enhance immunological effect against cancer, effect which has translated into clinical benefit. However, not all subjects are likely to respond to immunotherapies of this type.

The arrival of microarrays and molecular genomics is having a significant impact on the diagnostic capacity and prognostic classification of diseases, which can help predict the response of an individual patient to immunotherapies. For example, patent document EP3325653B1 defines a method for predicting the response of an individual to immunotherapies targeting immune checkpoints based on the use of molecular signatures.

Thus, in view of the state of the art, there is a need to identify biomarkers and alternative methods that allow predicting the response of subjects to immunotherapies, such as cancer patients, or of individuals susceptible to being treated therewith.

### DESCRIPTION OF THE INVENTION

The inventors have identified a set of genes, the expression levels of which allow predicting the response of a subject to immunotherapies, as well as identifying subjects who will respond to said immunotherapies.

Specifically, the inventors have demonstrated the association of elevated expression levels of 3 or more genes, from among *ABCA1, TMEM245, ATP13A3,* and *OLR1*, with improved survival outcomes in patients treated with immunotherapy. In fact, the inventors have demonstrated by means of a hazard ratio (HR) analysis that elevated expression levels of at least three genes, from among *ABCA1, TMEM245, ATP13A3,* and *OLR1*, preferably *ABCA1, TMEM245,* and *ATP13A3*, in subjects treated with immunotherapies, particularly immune checkpoint immunotherapies, is associated with a lower risk of having a fatal event over time (Table 2).

Thus, using the expression levels of these genes as molecular signatures allows predicting response to immunotherapies in subjects of interest, such as cancer patients or subjects susceptible to being treated with immunotherapies.

Therefore, the present invention provides an alternative approach for predicting the response of a subject to immunotherapies and for identifying subjects who will respond to said immunotherapies, having relevant clinical applications, such as the selection of responsive subjects and the identification of subjects who can be effectively treated with said immunotherapies, facilitating the application of personalized therapies.

On this basis, the inventors have developed *in vitro* methods for predicting the response of subjects to immunotherapies and for identifying subjects who will respond to said immunotherapies, as well as kits and uses for said purposes, which will be described in detail below.

### Methods of the invention

Based on the expression levels of the mentioned genes, the inventors have developed two applications, i.e., predicting the response of a subject to immunotherapies and identifying subjects who are likely to respond to said immunotherapies, giving rise to the two methods of the invention.

It should be noted that the methods of the invention that will be described below are applicable to any subject. In the present invention, the term "subject" or "individual", as it is used herein, refers to any animal, preferably a mammal, and includes, but is not limited to, domestic and farm animals, primates, and humans. In a preferred embodiment of the methods of the invention, the subject is a human being of any sex, age, or race. More preferably, the subject is a subject susceptible to being treated with immunotherapy. In another preferred embodiment, the subject may or may not have been previously treated with immunotherapy.

Furthermore, the sample in which the expression levels of genes are determined can be any biological sample originating or isolated from the subject. In that sense, in the present invention, a "sample" is understood to mean a part or a small amount of something which is considered representative of the total and which is taken or separated from it so as to be subjected to a study, analysis, or experimentation. Particularly, in the present invention, the term sample encompasses samples of biological origin, which are isolated from the subject including, without limitation to, blood samples and other fluid samples of biological origin, solid tissue samples, such as biopsy samples, or tissue cultures, or cells derived from same and their progeny, such as cells in cell culture, cell supernatants, cell lysates, serum, plasma, biological fluids, and tissue samples. The term "isolated" means that the biological sample has been separated or removed from the rest of the components naturally accompanying it. Techniques for obtaining biological samples from an individual are widely known in the state of the art, and any of these techniques can be used to put the present invention into practice.

That being said, in an aspect, the present invention relates to an *in vitro* method for predicting the response of a subject to an immunotherapy, hereinafter the "prediction method of the invention", wherein the method comprises the following steps:
a) determining the expression levels of at least 3 genes, wherein the genes are selected from the list consisting of: *ABCA1, TMEM245, ATP13A3,* and *OLR1*, in an isolated biological sample from the subject; and
b) comparing the expression levels of the genes determined in step a) with control values and detecting a significant deviation with respect to those control values, wherein said comparison and detection of the significant deviation allows predicting whether the subject will respond to said immunotherapy.

In a preferred embodiment, step a) comprises determining the expression levels of genes *ABCA1, TMEM245,* and *ATP13A3.*

In another embodiment, step a) comprises determining the expression levels of genes *ABCA1, TMEM245,* and *OLR1.*

In another embodiment, step a) comprises determining the expression levels of genes *ABCA1, ATP13A3,* and *OLR1.*

In another embodiment, step a) comprises determining the expression levels of genes *TMEM245, ATP13A3,* and *OLR1.*

In a preferred embodiment, alone or in combination with other preferred embodiments, the immunotherapy comprises, at least, one immune checkpoint inhibitor.

The term "predicting the response" is understood in the context of the present invention as determining whether or not the subject is likely to respond positively to a certain therapy or treatment, in the present invention, an immunotherapy.

As it is used herein, the term "will respond" refers to those subjects whose health condition is likely to improve, who are likely to recover from a disease or pathological condition, or to have a better prognosis if treated with the immunotherapy of interest.

In a first step [step a)], the prediction method of the invention comprises a) determining the expression levels of a set of genes in a biological sample isolated from the subject. Preferably, step a) comprises determining the expression levels of genes *ABCA1, TMEM245,* and *ATP13A3.*

The term "ABCA1" (NCBI Reference_NG_007981.1; HGNC:29, Ensembl version ENSG00000165029.17) refers to the human gene encoding an ABC transporter protein (ATP binding cassette subfamily A member 1). Particularly, it is known that said gene can encode the protein with accession number NP_005493.2.

The term "TMEM245" (HGNC:1363, Ensembl version ENSG00000106771.13) refers to the human gene encoding transmembrane protein 245. Particularly, it is known that said gene can encode different isoforms of this protein, the accession numbers of which are as follows: NP_114401.2, XP_011516748.1, XP_011516749.1, XP_011516751.1, XP_011516753.1, XP_011516754.1, XP_016870060.1, XP_016870061.1, XP_047279095.1, XP_047279096.1, or XP_047279098.1. The accession number of a transcript or an mRNA of said gene is NM_032012.4. In a particular embodiment of the invention, when the determined expression levels comprise the expression levels of the TMEM245 gene, the method comprises measuring the levels of the transcript with accession number NM_032012.4.

The term "ATP13A3" (HGNC:24113, Ensembl version ENSG00000133657.17) refers to the human gene encoding the ATPase 13A3 protein (ATPase 13A3). The ATPase 13A3 protein is a member of the P-type ATPase protein family which transports various cations across membranes. This gene is known to encode different isoforms of the ATPase 13A3 protein, for example, without being limiting, isoforms the accession numbers of which are NP_001354478.1, NP_078800.3, NP_001361765.1, XP_005269414.1, XP_011511423.1, or XP_011511425.1. The accession number of a transcript or an mRNA of said gene is NM_024524.4. In a preferred embodiment of the invention, when the determined expression levels comprise the expression levels of the ATPase 13A3 gene, the method comprises measuring the levels of the transcript with accession number NM_024524.4.

In a more preferred embodiment, alone or in combination with other preferred embodiments, step a) further comprises determining the expression levels of the *OLR1* gene.

The term "*OLR1*" (NCBI Reference Sequence NG_016743.2, HGNC:8133; Ensembl version ENSG00000173391.9) refers to the human gene encoding the oxidized low density lipoprotein receptor (known in the scientific literature as oxidized low density lipoprotein receptor 1). Said gene is known to encode different isoforms of this protein, the accession numbers of which are as follows: NP_002534.1, NP_001166103.1, NP_001166104.1, XP_047284863.1, XP_047284864.1, or XP_047284865.1.

In a more preferred embodiment, alone or in combination with other preferred embodiments, the expression levels determined in step a) comprise, or consist of, the expression levels of genes *OLR1*, *ABCA1, TMEM245,* and *ATP13A3.*

Another parameter that can be measured in combination with the expression levels described, preferably in combination with the expression levels of genes *ABCA1, TMEM245,* and *ATP13A3*, and more preferably in combination with the expression levels of genes *OLR1, ABCA 1, TMEM245,* and *ATP13A3,* are the expression levels of, at least, one, two, three, four, five, or six genes selected from the list consisting of *ITGAV, ANO6*, *CLEC5A, MSR1, CD80,* and *ADAM17.*

Thus, in a preferred embodiment, alone or in combination with other preferred embodiments, step a) further comprises determining the expression levels of at least one gene selected from the list consisting of: *ITGAV, ANO6*, *CLEC5A, MSR1*, *CD80, AOAM17,* and any combination thereof.

In another preferred embodiment, alone or in combination with other preferred embodiments, step a) further comprises determining the expression levels of, at least, two, three, four, five, or six genes selected from the list consisting of: *ITGAV, ANO6*, *CLEC5A, MSR1*, *CD80, ADAM17,* and any combination thereof.

The term "*ITGAV*" (HGNC:6150; Ensembl version ENSG00000138448.13) refers to the human gene encoding the integrin alpha-V protein (integrin subunit alphaV). Said gene can encode different isoforms of this protein, the accession numbers of which are as follows: NP_002201.2, NP_001138471.2, NP_001138472.2, XP_047300181.1. The accession number of a transcript or an mRNA of said gene is NM_002210.5. In a particular embodiment of the invention, when the determined expression levels comprise the expression levels of the *ITGAV* gene, the method comprises measuring the levels of the transcript with accession number NM_002210.5.

The term *"ANO6"* (NCBI Reference Sequence NG_028220.1; HGNC:25240, Ensembl version ENSG00000177119.17) refers to the human gene encoding the anoctamin 6 protein. Said gene can encode different isoforms of this protein, the accession numbers of which are as follows: NP_001020527.2, NP_001136150.1, NP_001136151.1, NP_001191732.1, NP_001397902.1, XP_005268764.1.

The term *"CLEC5A"* (HGNC:2054, Ensembl version ENSG00000258227.7) refers to the human gene encoding the CLEC5A protein (C-type lectin domain containing 5A). Said gene can encode different isoforms of this protein, the accession numbers of which are as follows: NP_037384.1, NP_001288096.1, XP_011514297.1. The accession number of a transcript or an mRNA of said gene is NM_013252.3. In a particular embodiment of the invention, when the determined expression levels comprise the expression levels of the *CLEC5A* gene, the method comprises measuring the levels of the transcript with accession number NM_013252.3.

The term "*MSR1*" (NCBI *Reference Sequence* NG_012102.1; HGNC:7376, Ensembl version ENSG00000038945.15) refers to the human gene encoding the MSR1 protein *(macrophage scavenger receptor 1).* Said gene can encode different isoforms of this protein, the accession numbers of which are as follows: NM_138715.3, NM_002445.4, NM_138716.3, NM_001363744.1, XM_024447161.2.

The term "*CD80*" (HGNC:1700; Ensembl version, ENSG00000121594.12) refers to the human gene encoding the CD80 protein (the accession number of which is NP_005182.1). The accession number of a transcript or an mRNA of said gene is NM_005191.4. In a particular embodiment of the invention, when the determined expression levels comprise the expression levels of the *CD80* gene, the method comprises measuring the levels of the transcript with accession number NM_005191.4.

The term "*ADAM17*" (NCBI Reference Sequence NG_029873.1; HGNC:195, Ensembl version ENSG00000151694.15) refers to the human gene encoding the ADAM 17 protein (ADAM metallopeptidase domain 17). This gene can encode different isoforms of this protein, the accession numbers of which are as follows: NP_003174.3, NP_001369706.1, NP_001369707.1, XP_047301566.1, XP_047301567.1, XP_047301568.1.

In a preferred embodiment, alone or in combination with other preferred embodiments, the expression levels comprise, or consist of, the expression levels of genes *OLR1*, *ABCA1, TMEM245, ATP13A3, ITGAV, ANO6*, and *CLEC5A.*

In a preferred embodiment of the prediction method of the invention, alone or in combination with the other preferred embodiments, the mean of the expression levels of all the genes determined in a) are calculated.

As understood by a person skilled in the art, gene expression levels can be determined by means of standard methodologies well known in the state of the art, and any of said methodologies can be used in the context of the present invention. Determining the expression levels of the genes used in the present invention may comprise measuring the mRNA levels and/or the complementary DNA (cDNA) levels.

By way of illustration and without limitation, the mRNA levels of said genes can be quantified by means of using conventional methods, for example, methods comprising mRNA amplification and quantification of the amplification product of said mRNA, such as electrophoresis and staining, or alternatively, by means of *Southern blot* and the use of suitable probes, *Northern blot* and the use of specific mRNA probes for the gene or genes of interest, or the corresponding cDNA thereof, hybridization, real-time quantitative PCR (or RT-Q-PCR), microarrays and RNA sequencing, etc.

It is contemplated that the expression levels of the genes used in the invention can be determined by means of evaluating circulating DNA. Methods and techniques for this purpose are known in the state of the art including, but are not limited to, exome sequencing. Another possible technique to determine the expression levels of the genes used in the invention is digital PCR (dPCR).

Determining the expression levels of the genes used in the present invention may also comprise measuring the levels of protein encoded by said genes. In this case, the sample should be treated to extract proteins. Methods for extracting or isolating proteins are known to the person skilled in the art and are commercially available. The levels of the gene encoding the protein of interest can be quantified by means of any conventional method which allows detecting and quantifying said protein in a sample from a subject. By way of illustration and not limitation, the levels of said protein can be quantified, for example, by means of using antibodies with the capacity to bind specifically to the protein of interest and subsequently quantifying the complexes formed.

Once the gene expression levels are determined in step (a), the prediction method of the invention comprises comparing said levels with control values [step b)].

In the present invention, the term "control values" refers to values or expression levels of the genes of step a) that are taken as reference, and are suitable for establishing whether or not the expression levels of the genes determined in a) are elevated with respect to said values or levels.

In a preferred embodiment, alone or in combination with other preferred embodiments, the control values are the expression levels of the genes of step a) but determined in a healthy subject, or the mean expression levels of the genes of step a) in a cohort of healthy subjects. In another alternative embodiment, the control values are the expression levels of the genes of step a) but determined in a subject who has not responded to the immunotherapy, or the mean expression levels of the genes of step a) in a cohort of subjects who have not responded to the immunotherapy. Methods for determining gene expression levels have been described in the preceding paragraphs.

Lastly, based on the comparison of the expression levels of the genes determined in a) with control values and the detection of a significant deviation from those control values, it can be known whether a subject will respond to said immunotherapy.

"Significant deviation" is understood to mean that the expression levels determined are elevated with respect to the control values, preferably elevated by at least 1 fold, 1.1 folds, 1.2 folds, 1.3 folds, 1.4 folds, 1.5 folds, 1.6 folds, 1.7 folds, 1.8 folds, 1.9 folds, 2 folds, or more with respect to the control values. In that sense, detecting said significant deviation indicates that the subject will respond to the immunotherapy.

On the other hand, the inventors have developed a method for identifying subjects who are likely to respond to immunotherapies based on the association of elevated expression levels of 3 or more genes, from among *ABCA1, TMEM245, ATP13A3,* and *OLR1*, with improved survival outcomes in patients treated with immunotherapy.

Thus, in another aspect, the present invention relates to an *in vitro* method for identifying a subject who will respond to an immunotherapy, hereinafter the "identification method of the invention", which comprises:
a) determining the expression levels of at least 3 genes, wherein the genes are selected from the list consisting of: *ABCA1, TMEM245, ATP13A3,* and *OLR1*, in an isolated biological sample from the subject; and
b) comparing the expression levels of the genes determined in step a) with control values, and detecting a significant deviation with respect to those control values, wherein detecting said significant deviation allows identifying a subject who will respond to said immunotherapy.

In a preferred embodiment, step a) comprises determining the expression levels of genes *ABCA1, TMEM245,* and *ATP13A3.*

In another embodiment, step a) comprises determining the expression levels of genes *ABCA1, TMEM245,* and *OLR1.*

In another embodiment, step a) comprises determining the expression levels of genes *ABCA1, ATP13A3,* and *OLR1.*

In another embodiment, step a) comprises determining the expression levels of genes *TMEM245, ATP13A3,* and *OLR1.*

In a preferred embodiment, alone or in combination with other preferred embodiments, the immunotherapy comprises, at least, one immune checkpoint inhibitor.

The expression "is likely to respond" or "will respond", as it is used herein, refers to the fact that the subject's health condition is likely to improve, that the subject is likely to recover from a disease or pathological condition, or to have a better prognosis if treated with the immunotherapy of interest.

The present method is applicable for selecting patients for clinical trials or for routine clinical practice. Furthermore, identification of subjects who will respond to the immunotherapy is indicative of the fact that said subjects can be effectively treated with said immunotherapy, and can guide the selection of therapies in a personalized manner in said subject.

The terms used to define the present inventive aspect have been explained above in the prediction method of the invention, and both, the terms and their preferred embodiments are applicable to the present aspect of the invention.

The identification method of the invention comprises a first step intended for determining the expression levels of a set of genes, as mentioned above, in an isolated biological sample from the subject. Preferably, step a) comprises determining the expression levels of genes *ABCA1, TMEM245,* and *ATP13A3.* Once said levels are determined, they are compared with control values in a second step. The term "control values" has been defined above, and both, the term and its preferred embodiments are applicable to the present aspect of the invention. Comparing and detecting a significant deviation allows identifying subjects who will respond or are likely to respond to said immunotherapy.

"Significant deviation" is understood to mean that the expression levels determined are elevated with respect to the control values, preferably elevated by, at least, 1 fold, 1.1 folds, 1.2 folds, 1.3 folds, 1.4 folds, 1.5 folds, 1.6 folds, 1.7 folds, 1.8 folds, 1.9 folds, 2 folds, or more with respect to the control values. In that sense, detecting said significant deviation allows identifying a subject who will respond to said immunotherapy.

In a more preferred embodiment, alone or in combination with other preferred embodiments, step a) further comprises determining the expression levels of the *OLR1* gene.

In a more preferred embodiment, alone or in combination with other preferred embodiments, the expression levels determined in step a) comprise, or consist of, the expression levels of genes *OLR1*, *ABCA1, TMEM245,* and *ATP13A3.*

Another parameter that can be measured in combination with the expression levels described, preferably in combination with the expression levels of genes *ABCA1, TMEM245,* and *ATP13A3,* and more preferably in combination with the expression levels of genes *OLR1, ABCA 1, TMEM245,* and *ATP13A3,* are the expression levels of, at least, one, two, three, four, five, or six genes selected from the list consisting of *ITGAV, ANO6*, *CLEC5A, MSR1, CD80,* and *ADAM17.*

Thus, in a preferred embodiment, alone or in combination with other preferred embodiments, step a) further comprises determining the expression levels of, at least, one gene selected from the list consisting of: *ITGAV, ANO6*, *CLEC5A, MSR1*, *CD80, ADAM17,* and any combination thereof.

In another preferred embodiment, alone or in combination with other preferred embodiments, step a) further comprises determining the expression levels of at least two, three, four, five, or 6 genes selected from the list consisting of: *ITGAV, ANO6*, *CLEC5A, MSR1*, *CD80, ADAM17,* and any combination thereof.

In a preferred embodiment, alone or in combination with other preferred embodiments, the expression levels comprise, or consist of, the expression levels of genes *OLR1*, *ABCA1, TMEM245, ATP13A3, ITGAV, ANO6*, and *CLEC5A.*

In a preferred embodiment of the identification method of the invention, alone or in combination with other preferred embodiments, the mean of the expression levels of all the genes determined in a) are calculated.

In the present invention, the term "immunotherapy" can be defined as a set of strategies intended for inducing, stimulating, or activating the immune system. In the context of immunotherapies targeting cancer, immunotherapy is intended for activating the immune system.

Immunotherapies include, among others, immune checkpoint inhibitors. Immune checkpoints are known in the art (Naidoo et al. British Journal of Cancer (2014) 111, 2214-2219; Pardoll, Nature 2012, vol. 12, 252-264) and the term is well understood in the context of cancer therapy. The best known are CTLA-4, PD-1 and its ligand PDL-1. Others include TIGIT (for example, without being limiting, tiragolumab), TIM-3, KIR, LAG-3 (such as, for example, without being limiting, relatlimab), VISTA, BTLA, or formulations thereof, which contain the same compounds in a different pharmaceutical formulation (for example opdualag: nivolumab/relatlimab combination). Immune checkpoint inhibitors inhibit normal immunosuppressive function, for example by reducing the expression of checkpoint molecules or by binding to said molecules and blocking normal receptor/ligand interactions. Since immune checkpoints slow down the immune system's response to an antigen, an immune checkpoint inhibitor reduces this immunosuppressive effect and enhances immune response. Immune checkpoint inhibitors are known in the art, such as anti-CTLA-4 antibodies and anti-PD-1/L1 antibodies.

Immune checkpoint inhibitors can be proteins, peptides, peptidomimetics, peptoids, antibodies, antibody fragments, small inorganic molecules, small non-nucleic organic molecules, or nucleic acids such as antisense nucleic acids, small interfering RNA (siRNA) molecules, or oligonucleotides, among others.

Preferably, immune checkpoint inhibitors are antibodies, more particularly antibodies that bind to molecules constituting immune checkpoints, where both receptors and ligands thereof may be included.

As it has been mentioned, the expression levels of genes *ABCA1, TMEM245,* and *ATP13A3,* and more preferably of genes *OLR1*, *ABCA1, TMEM245,* and *ATP13A3,* are useful in predicting the response of a subject to immunotherapies, as well as in identifying subjects who will respond to said immunotherapies, and therefore are susceptible to being effectively treated with said immunotherapies. In fact, the inventors have demonstrated that the use of said expression levels allows predicting whether a subject will respond to immunotherapies based on immune checkpoint inhibitors such as anti-PD(L)1 or anti-CTLA-4 therapies, both alone and in combination. Furthermore, since these genes are highly expressed in regulatory T cells (Tregs), expression levels may also be useful in predicting the response of a subject to immunotherapies targeting regulatory T-cells or Tregs, as well as in identifying subjects who will respond to said immunotherapies.

Thus, in a preferred embodiment of any of the methods of the invention, alone or in combination with other preferred embodiments, immunotherapy comprises a regulatory T-cells (Tregs)-targeted therapy. Since the identified genes are associated with high expression of Tregs, the described signature can be used to predict response to therapies against this immunological population.

In a more preferred embodiment, the Tregs-targeted therapy comprises, at least, one inhibitor or agent targeting TIGIT, preferably wherein the inhibitor or agent targeting TIGIT is selected from the list consisting of tiragolumab, domvanalimab, ociperlimab, and any combination thereof, and/or at least one inhibitor or agent targeting OX40, for example, MEDI6469, among others.

In a preferred embodiment of any of the methods of the invention, alone or in combination with other preferred embodiments, the immunotherapy comprises at least one immune checkpoint inhibitor selected from the list consisting of a PD-1 inhibitor, a PDL-1 inhibitor, a CTLA-4 inhibitor, a TIGIT inhibitor, and any combination thereof.

In a more preferred embodiment of any of the methods of the invention, alone or in combination with other preferred embodiments, the immunotherapy comprises a PD-1 inhibitor and/or a PDL-1 inhibitor and/or a CTLA-4 inhibitor.

PD-1 (Programmed Cell Death Protein 1), also known as CD279, is a cell surface receptor that belongs to the immunoglobulin superfamily. PD-1 is a member of the CD28 family of T-cell regulators and is expressed on T-cells, B-cells, and macrophages. It binds to the ligands PD-L1 (also known as B7 homologue) and PD-L2 (also known as B7-DC). Its structure includes an extracellular IgV domain, a transmembrane region, and an intracellular tail containing two phosphorylation sites. Known as an immune checkpoint protein, PD-1 works as an inducible immune-modulator receptor which plays a role, for example, in the downregulation of T-cell responses to antigen stimulation.

PD-L1 (Programmed Death-ligand 1) is the predominant ligand for PD-1. The binding of PD-L1 to PD-1 inhibits T-cell activity, reducing cytokine production and suppressing T-cell proliferation. Cancer cells expressing PD-L1 are capable of exploiting this mechanism to inactivate T-cell anti-tumor activity by means of binding PD-L1 to the PD-1 receptor.

In view of their immune response regulating properties, PD-1 and PDL-1 have been investigated as a potential target for immunotherapy, including the treatment of cancer and autoimmune diseases.

Those agents intended for blocking the binding of PD-1 or PDL-1 such as, for example, monoclonal antibodies, constitute PD-1 or PDL-1 inhibitors (referred to collectively as PD-1/L1 inhibitors), being immune checkpoints inhibitors.

Examples of inhibitors or agents targeting PD-1/L1 include, but are not limited to, lambelimumab, nivolumab, lambrolozumab, pidilizumab, pembrolizumab, atezolizumab, durvalumab, avelumab, cemiplimab, or dostarlimab. Thus, in a more preferred embodiment, alone or in combination with other preferred embodiments, the PD-1/L1 inhibitor is selected from the list consisting of lambelimumab, nivolumab, lambrolozumab, pidilizumab, pembrolizumab, atezolizumab, durvalumab, avelumab, cemiplimab, and dostarlimab.

CTLA-4 (cytotoxic T-lymphocyte associated protein or antigen 4), also known as CD152, is a member of the immunoglobulin protein superfamily. CTLA-4 acts by regulating T-cell activation and maintaining immune homeostasis, being a molecule that reduces T-cell function under certain circumstances. Therefore, inhibitors or agents that block CTLA-4 release this arrest mechanism and increase T-cell activation. Thus, CTLA-4 is a prominent immune checkpoint that can be exploited by immunotherapies.

Examples of inhibitors or agents targeting CTLA-4 include, but are not limited to, ipilimumab or tremelimumab. In a more preferred embodiment, alone or in combination with other preferred embodiments, the CTLA-4 inhibitor is selected from the list consisting of ipilimumab and tremelimumab.

TIGIT (T-cell immunoglobulin and ITIM domain) is an emerging immune checkpoint that inhibits immune cell responses in multiple stages of the cancer-immunity cycle. TIGIT prevents dendritic cells from preparing T-cells, prevents natural killer cells and cytotoxic T-cells from killing tumor cells, and enhances the immunosuppressive activity of regulatory T-cells. Accordingly, TIGIT constitutes an important target in immunotherapy-based cancer treatments.

Examples of inhibitors or agents targeting TIGIT include, but are not limited to, tiragolumab, domvanalimab, ociperlimab. In a more preferred embodiment, alone or in combination with other preferred embodiments, the TIGIT inhibitor is selected from the list consisting of tiragolumab, domvanalimab, and ociperlimab.

In a preferred embodiment of any of the methods of the invention, alone or in combination with other preferred embodiments, the subject has cancer.

The term "cancer" can be defined as a broad group of diseases characterized by the abnormal and uncontrolled rapid proliferation of cells in a subject.

Examples of types of cancer include, but are not limited to, breast cancer, melanoma, bladder cancer, esophageal adenocarcinoma, glioblastoma, hepatocellular carcinoma, head and neck squamous cell carcinoma, non-small cell lung cancer, small cell lung cancer, gastric cancer and urothelial cancer.

In a preferred embodiment of any of the methods of the invention, alone or in combination with other preferred embodiments, the isolated biological sample is from peripheral blood.

In another preferred embodiment of any of the methods of the invention, alone or in combination with other preferred embodiments, the isolated biological sample is from a tumor.

Thus, in a more preferred embodiment, the cancer is selected from the list consisting of breast cancer, melanoma, bladder cancer, esophageal adenocarcinoma, glioblastoma, hepatocellular carcinoma, head and neck squamous cell carcinoma, non-small cell lung cancer, small cell lung cancer, gastric cancer and urothelial cancer.

In the present invention, "tumor" is understood to mean any growth of a tissue due to the uncontrolled proliferation of cells. The tumor can be benign or malignant. A tumor is considered benign when the cells that form the tumor do not invade other tissues or cause metastases in other parts of the body. Normally, the benign tumor is well encapsulated and the cells show no structural changes. On the contrary, a tumor is considered malignant when the cells that form the tumor can grow rapidly, present anaplasia in a conventional manner, and are capable of invading adjacent tissues, and even spread to other parts of the body, a process known as metastasis.

Examples of the types of tumors in the context of the present invention include, but are not limited to, breast cancer tumor, malignant tumor of the melanocytes, bladder tumor, esophageal adenocarcinoma tumor, glioblastoma tumor, hepatocellular carcinoma tumor, head and neck squamous cell carcinoma tumor, non-small cell lung cancer tumor, small cell lung cancer tumor, gastric tumor, and urothelial tumor.

Thus, in a more preferred embodiment, the tumor is selected from the list consisting of breast cancer tumor, malignant tumor of the melanocytes, bladder tumor, esophageal adenocarcinoma tumor, glioblastoma tumor, hepatocellular carcinoma tumor, head and neck squamous cell carcinoma tumor, non-small cell lung cancer tumor, small cell lung cancer tumor, gastric tumor, and urothelial tumor.

In another more preferred embodiment of any of the methods of the invention, alone or in combination with other preferred embodiments, when the isolated biological sample is from a tumor, the tumor comprises elevated levels of Treg lymphocytes.

The levels of Tregs lymphocytes in a sample can be evaluated or quantified by means of methods and techniques known to a person skilled in the art such as, without being limiting to, immunohistochemistry or immunofluorescence with markers such as CD3/FOXP3, CD25, CTLA-4, and GITR. Their levels can also be determined by means of flow cytometry using FOXP3 labeling and isolation of the population of interest by means of cell sorting (FACS, Fluorescence-activated cell sorting). A person skilled in the art can determine whether or not the sample has elevated levels of Tregs by means of comparing with reference values. Furthermore, Tregs identification and characterization methods are described in Epple, HJ et al. (2006). Mucosal but not peripheral FOXP3+ regulatory 7 cells are highly increased in untreated HIV infection and normalize after suppressive HAART. Blood, 108(9), 3072-3078.

### Uses of the invention

As explained at the beginning of this description, the expression levels of the genes mentioned in the preceding aspects are useful for predicting the response of a subject to immunotherapies, as well as for identifying subjects who will respond to said immunotherapies. Therefore, in the present invention, uses of said expression levels are also contemplated as inventive aspects.

Thus, in another aspect, the invention relates to the *in vitro* use of the expression levels of, at least, 3 genes, wherein the genes are selected from the list consisting of: *ABCA1, TMEM245, ATP13A3*, and *OLR1*, to predict the response of a subject to immunotherapy, hereinafter, "use for predicting response to immunotherapies of the invention", wherein elevated expression levels with respect to control values, preferably elevated by, at least, 1 fold, 1.1 folds, 1.2 folds, 1.3 folds, 1.4 folds, 1.5 folds, 1.6 folds, 1.7 folds, 1.8 folds, 1.9 folds, 2 folds, or more with respect to the control values, indicate that the subject will respond to the immunotherapy.

The terms used to define the present inventive aspect have been explained above in the prediction method of the invention, and both, the terms and their preferred embodiments, are applicable to the present aspect of the invention.

In a preferred embodiment, alone or in combination with other preferred embodiments, the immunotherapy comprises, at least, one immune checkpoint inhibitor.

A preferred embodiment provides the use for predicting response to immunotherapies of the invention, wherein the genes are *ABCA1, TMEM245,* and *ATP13A3.*

Another preferred embodiment provides the use for predicting response to immunotherapies of the invention, wherein the genes are *ABCA1, TMEM245,* and *OLR1.*

Another preferred embodiment provides the use for predicting response to immunotherapies of the invention, wherein the genes are *ABCA1, ATP13A3*, and *OLR1.*

Another preferred embodiment provides the use for predicting response to immunotherapies of the invention, wherein the genes are *TMEM245, ATP13A3,* and *OLR1.*

In a preferred embodiment, alone or in combination with other preferred embodiments, the expression levels comprise, or consist of, the expression levels of genes *OLR1*, *ABCA1, TMEM245,* and *ATP13A3*, wherein elevated or increased expression levels with respect to control values indicate that the subject will respond to the immunotherapy.

As for the methods of the invention described above, the expression levels of at least one, two, three, four, five, or six genes selected from the list consisting of *ITGAV, ANO6*, *CLEC5A, MSR1*, *CD80,* and *ADAM17,* can be used in combination with the expression levels of *ABCA1, TMEM245,* and *ATP13A3*, and more preferably in combination with genes *OLR1*, *ABCA1, TMEM245,* and *ATP13A3.*

Thus, in a preferred embodiment, alone or in combination with other preferred embodiments, the expression levels further comprise the expression levels of, at least, one gene selected from the list consisting of: *ITGAV, ANO6*, *CLEC5A, MSR1*, *CD80, AOAM17,* and any combination thereof, wherein elevated or increased expression levels with respect to control values indicate that the subject will respond to the immunotherapy.

As for the prediction method of the invention, there is also contemplated the use of the expression levels of at least two, three, four, five, or six genes selected from the list consisting of *ITGAV, ANO6*, *CLEC5A, MSR1, CD80,* and *ADAM17.* Thus, the different combinations of genes, the expression levels of which are determined, also apply to the present aspect of the invention.

In a preferred embodiment, alone or in combination with other preferred embodiments, the expression levels comprise, or consist of, the expression levels of genes *OLR1*, *ABCA1, TMEM245, ATP13A3, ITGAV, ANO6*, and *CLEC5A,* wherein elevated or increased expression levels with respect to control values indicate that the subject will respond to the immunotherapy.

Another aspect of the present invention relates to the *in vitro* use of the expression levels of at least 3 genes, wherein the genes are selected from the list consisting of: *ABCA1, TMEM245, ATP13A3,* and *OLR1*, to identify a subject who will respond to immunotherapy, hereinafter the "use for identifying subjects of the invention", wherein elevated expression levels with respect to control values, preferably elevated by at least 1 fold, 1.1 folds, 1.2 folds, 1.3 folds, 1.4 folds, 1.5 folds, 1.6 folds, 1.7 folds, 1.8 folds, 1.9 folds, 2 folds, or more with respect to the control values, allows identifying a subject who will respond to immunotherapy.

A preferred embodiment provides the use for identifying subjects of the invention, wherein the genes are *ABCA1, TMEM245,* and *ATP13A3.*

Another preferred embodiment provides the use for identifying subjects of the invention, wherein the genes are *ABCA1, TMEM245,* and *OLR1.*

Another preferred embodiment provides the use for identifying subjects of the invention, wherein the genes are *ABCA1, ATP13A3,* and *OLR1.*

Another preferred embodiment provides the use for identifying subjects of the invention, wherein the genes are *TMEM245, ATP13A3,* and *OLR1.*

The terms used to define the present inventive aspect have been explained above for the methods of the invention, and both, the terms and their preferred embodiments are applicable to the present aspect of the invention.

In a preferred embodiment, alone or in combination with other preferred embodiments, the immunotherapy comprises, at least, one immune checkpoint inhibitor.

In a preferred embodiment, alone or in combination with other preferred embodiments, the expression levels comprise, or consist of, the expression levels of genes *OLR1*, *ABCA1, TMEM245,* and *ATP13A3*, wherein elevated or increased expression levels with respect to control values allows identifying a subject who will respond to immunotherapy.

As for the methods of the invention described above, the expression levels of at least one, two, three, four, five, or six genes selected from the list consisting of *ITGAV, ANO6*, *CLEC5A, MSR1*, *CD80,* and *ADAM17,* can be used in combination with the expression levels of genes *ABCA1, TMEM245,* and *ATP13A3,* and more preferably, in combination with genes *OLR1*, *ABCA1, TMEM245,* and *ATP13A3.*

In that sense, in a preferred embodiment, alone or in combination with other preferred embodiments, the expression levels further comprise the expression levels of, at least, one gene selected from the list consisting of: *ITGAV, ANO6*, *CLEC5A, MSR1*, *CD80, AOAM17,* and any combination thereof, wherein elevated or increased expression levels with respect to control values allows identifying a subject who will respond to immunotherapy.

As for the identification method of the invention, there is also contemplated the use of the expression levels of, at least, two, three, four, five, or six genes selected from the list consisting of *ITGAV, ANO6*, *CLEC5A, MSR1, CD80,* and *ADAM17.* Thus, the different combinations of genes, the expression levels of which are determined, also apply to the present aspect of the invention.

In a preferred embodiment, alone or in combination with other preferred embodiments, the expression levels comprise, or consist of, the expression levels of genes *OLR1*, *ABCA1, TMEM245, ATP13A3, ITGAV, ANO6*, and *CLEC5A,* wherein elevated or increased expression levels with respect to control values allows identifying a subject who will respond to immunotherapy.

In a preferred embodiment of any of the uses of the invention, alone or in combination with other preferred embodiments, immunotherapy comprises a Tregs-targeted therapy.

In another preferred embodiment of any of the uses of the invention, alone or in combination with other preferred embodiments, the immunotherapy comprises, at least, one immune checkpoint inhibitor selected from the list consisting of a PD-1 inhibitor, a PDL-1 inhibitor, a CTLA-4 inhibitor, a TIGIT inhibitor, and any combination thereof.

In a more preferred embodiment, alone or in combination with other preferred embodiments, the immunotherapy comprises a PD-1 inhibitor and/or a PDL-1 inhibitor and/or a CTLA-4 inhibitor.

As mentioned, the terms used to define the uses of the invention have been explained above for the methods of the invention, and both, the terms and their preferred embodiments are applicable to the uses of the invention.

### Kit of the invention

The uses and methods of the invention are put into practice based on determination of the expression levels of, at least, 3 genes, wherein the genes are selected from the list consisting of: *ABCA1, TMEM245, ATP13A3,* and *OLR1*, preferably genes *ABCA1, TMEM245, ATP13A3,* and more preferably on determination of genes *OLR1*, *ABCA1, TMEM245,* and *ATP13A3.* The means employed for this determination of the expression levels may be part of a kit.

Thus, another aspect of the present invention relates to a kit, hereinafter the "kit of the invention", comprising means for the *in vitro* determination of the expression levels of, at least, 3 genes, wherein the genes are selected from the list consisting of: *ABCA1, TMEM245, ATP13A3* and *OLR1*, preferably means for the *in vitro* determination of the expression levels of genes *ABCA1, TMEM245,* and *ATP13A3,* and more preferably means for the *in vitro* determination of the expression levels of genes *OLR1*, *ABCA1, TMEM245* and *ATP13A3.*

As is known to a person skilled in the art, gene expression levels can be determined by means of techniques and methods known in the state of the art, as explained in the preceding aspects of the invention, and both the techniques and their preferred embodiments are applicable to the kit of the invention.

In addition, the kit of the invention may comprise means for the *in vitro* determination of the expression levels of at least one, two, three, four, five, or six genes selected from the list consisting of *ITGAV, ANO6*, *CLEC5A, MSR1, CD80,* and *AOAM17,* and any combination thereof.

In a preferred embodiment, the kit of the invention comprises means for the *in vitro* determination of the expression levels of genes *OLR1*, *ABCA1, TMEM245, ATP13A3, ITGAV, ANO6*, and *CLEC5A.*

Furthermore, as understood by a person skilled in the art, the kit may comprise other components useful in putting the present invention into practice such as buffer solutions, delivery vehicles, material supports, positive and/or negative control components, etc. In addition to the mentioned components, the kits may also include instructions for practicing the object of the invention. These instructions may be present in the mentioned kits in different ways, one or more of which may be present in the kit. One way in which these instructions may be present is as information printed on a suitable medium or substrate, for example, a sheet or sheets of paper on which the information is printed, in the kit packaging, in a package insert, etc. Another medium would be a computer-readable medium, for example, a CD, a USB, etc., in which the information has been recorded. Another medium that may be present is a website address that can be used over the Internet to access the information at a remote site. Any suitable medium may be present in the kits.

Furthermore, the kit of the invention is useful for predicting the response of a subject to immunotherapies and for identifying subjects who will respond to immunotherapy, and therefore it allows identifying subjects susceptible to being effectively treated with said immunotherapies and can be used in any of the methods of the invention that have been described above.

Thus, in another aspect, the invention relates to the use of the kit of the invention in any of the methods of the invention.

In another aspect, the invention relates to the use of the kit of the invention for predicting *in vitro* the response of a subject to immunotherapy, preferably wherein said immunotherapy comprises, at least, one immune checkpoint inhibitor.

In another aspect, the invention relates to the *in vitro use* of the kit of the invention for identifying subjects who will respond to immunotherapy, preferably wherein said immunotherapy comprises, at least, one immune checkpoint inhibitor.

The terms used to define the kit and uses of the kit of the invention have been explained for the preceding aspects of the invention, and both the terms and their preferred embodiments are applicable to different uses of the kit of the invention.

### DESCRIPTION OF THE FIGURES

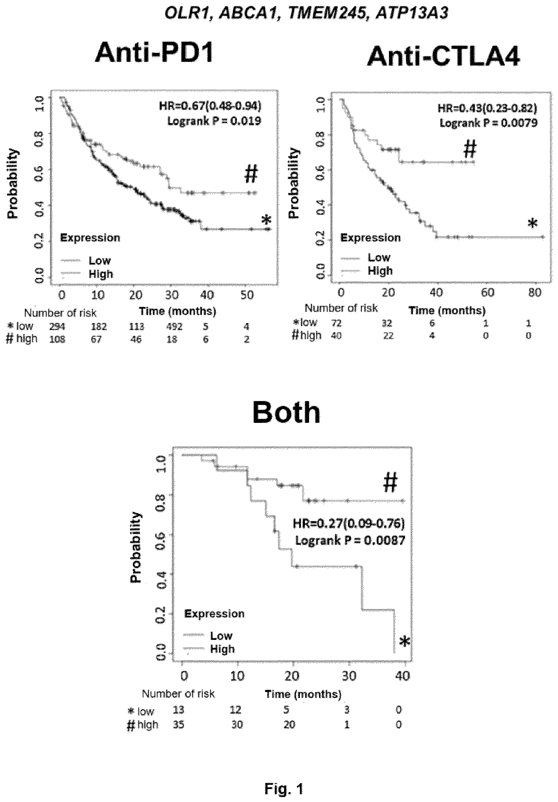

**Fig. 1****.** It shows the Kaplan-Meier survival plots of the association between the mean expression levels (as genetic signature) of selected surface genes OLR1, ABCA1, TMEM245, and ATP13A3 and the prognosis of patients with anti-PD1 treatment, anti-CTLA4 treatment, and both treatments.

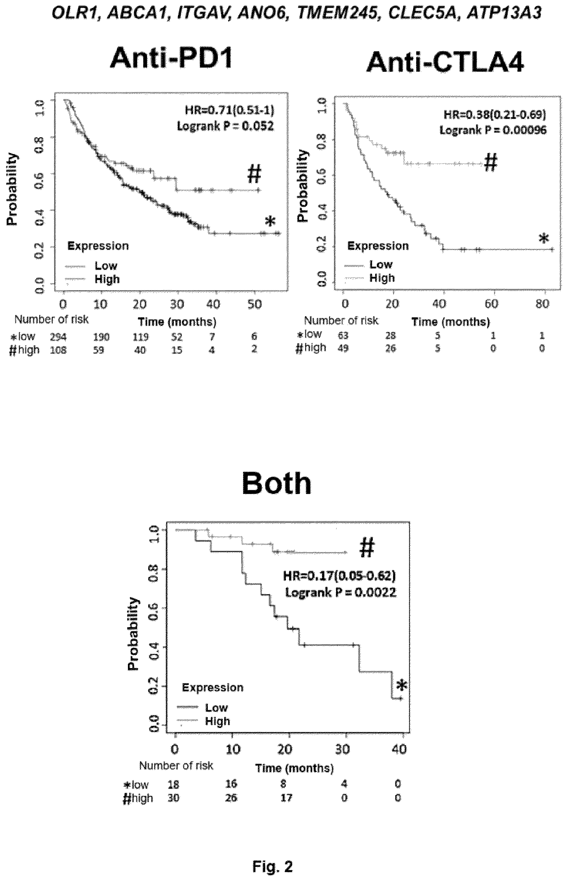

**Fig. 2****.** It depicts the Kaplan-Meier survival plots of the association between the mean expression levels (as genetic signature) of selected surface genes OLR1, ABCA1, TMEM245, ATP13A3, ITGAV, ANO6, and CLEC5A and the prognosis of patients with anti-PD1 treatment, anti-CTLA4 treatment, and both treatments.

### EXAMPLES

Next, the invention will be illustrated by means of assays carried out by the inventors, evidencing the invention.

### Methods

Breast cancer samples were used which included patients from the datasets previously described in Lanczky A, et al. Web-Based Survival Analysis Tool Tailored for Medical Research (KMplot): Development and Implementation. J Med Internet Res. 26 July 2021;23(7):e27633 y Györffy B, et al. An online survival analysis tool to rapidly assess the effect of 22,277 genes on breast cancer prognosis using microarray data of 1,809 patients. Breast cancer research and treatment. 2010; 123(3), 725-731. Doi.org/10.1007/s10549-009-0674-9 as a cohort to identify genes the expression of which was correlated with high Treg infiltration.

Immune cell infiltration in each tumor sample was determined using the normalized RNA-seq-based transcriptomic gene expression data as input for the signature and the xCell algorithm described in Aran D., Hu Z., Butte AJ. xCell: digitally portraying the tissue cellular heterogeneity landscape. Genome Biol. 2017;18(1):220. Doi:10.1186/s13059-017-1349-1. xCell is designed to calculate surrogate markers of cell proportions for sets of sixty-four different cell types. Subsequently, for each gene, Spearman's rank correlation was calculated with each gene to compare its normalized gene expression and xCell-derived Treg infiltration scores for regulatory T-cells. In other words, a high Treg score corresponds to a higher proportion of Treg cells among all cells in the entire tumor sample. Lastly, all investigated genes were classified based on the Spearman's correlation coefficients obtained.

Once the genes related to high expression of Tregs have been identified using data collected in the *in silico* human surfaceome described in Bausch-Fluck D, et al. The in silico human surfaceome. Proc Natl Acad Sci USA. 2018;115(46):E10988-E10997, genes encoding surface proteins were identified. This public biomedical resource can be used to filter multiomic data and discover cell phenotypes and new surfaceome markers.

10 upregulated genes encoded proteins expressed in the cell membrane, eight of them (7.8%) being of the HER2 subtype, one (0.7%) in Luminal A, and three (4.9%) genes of the basal-like subtype. From these 10 genes, different genetic signatures were selected for analysis.

The Tumor Immune Estimation Resource (TIMER) platform described in Li T., et al. TIMER: A Web Server for Comprehensive Analysis of Tumor-Infiltrating Immune Cells. Cancer Res. 2017;77(21):e108-e110. Doi:10.1158/0008-5472.CAN-17-0307 analyzes the association between the presence of tumor immune infiltrates (CD4+ T-cells, CD8+ T-cells, macrophages, neutrophils, and B-cells) and the expression of selected genes. TIMER contains 10,897 samples of various cancer types from The Cancer Genome Atlas (TCGA) project and provides abundances of immune infiltrates estimated by means of various immune deconvolution methods. TIMER applies a deconvolution method that was previously published by Li B., et al. Comprehensive analyses of tumor immunity: implications for cancer immunotherapy. Genome biology. 2016; 17(1), 174. Doi.org/10.1186/s13059-016-1028-7 to infer the abundance of tumor-infiltrating immune cells from gene expression profiles. Tumor immune infiltrates in breast cancer subtypes were explored.

Using public genomic data from various sources and described in Lanczky A., *et al.* (Lánczky A, Györffy B. Web-Based Survival Analysis Tool Tailored for Medical Research (Kmplot): Development and Implementation. J Med Internet Res. 26 July 2021;23(7):e27633) as well as from the Cancer Genome Atlas (TCGA) (cBioPortal for Cancer Genomics), gene expression and survival were correlated following a Kaplan-Meier analysis, as described in Lanczky A., *et al*., in a combined cohort of patients treated with immunotherapy for different types of tumors, including bladder tumor (n=90), esophageal adenocarcinoma tumor (n=103), glioblastoma tumor (n=28), hepatocellular carcinoma tumor (n=22), HNSCC tumor (n=110), melanoma tumor (n=570), NSCLC tumor (n=21), NSLC tumor (n=22), breast tumor (n=14), gastric tumor (n=45), and urothelial tumor (n=392). As mentioned, the described data was obtained by means of identification in Gene Expression Omnibus (GEO) using the keywords "gene expression", "PD1", "CTLA4", and "immunotherapy", as well as the names of available immunotherapy agents. In this cohort, the correlation with overall survival (OS) alone was evaluated and patients were also separated into two cohorts according to the best cut-off values. Depending on the therapy administered, the anti-PD1 treatment included n = 797 samples and the anti-CTLA4 cohort included n = 131 samples.

### Results

Evaluation was performed on the expression levels of genes MSR1 (HGNC:7376, Ensembl: ENSG00000038945), CD80 (HGNC:1700, Ensembl: ENSG00000121594), OLR1 (HGNC:8133, Ensembl:ENSG00000173391), ABCA1 (HGNC:29, Ensembl:ENSG00000165029, ITGAV (HGNC:6150, Ensembl:ENSG00000138448), ADAM17 (HGNC:195, Ensembl:ENSG00000151694), ANO6 (HGNC:25240, Ensembl:ENSG00000177119), TMEM245 (HGNC:1363, Ensembl:ENSG00000106771), CLEC5A (HGNC:2054, Ensembl:ENSG00000258227), ATP13A3 (HGNC:24113, Ensembl:ENSG00000133657) individually and in combinations, as genetic signatures.

Table 1 shows the individual results of hazard ratio as a function of time for each of the genes in patients treated with anti-PD1 and anti-CTLA4 therapies, in which it is observed how the risk of having an event such as death over time is lower in those patients with the presence of the gene. This risk is statistically significant with p-values of ≤0.05.

**Table 1. Individual results of hazard ratio (HR) of each of the genes. The significance of the values is indicated as low significant risk factor; low sig. RF, high significant risk factor; high sig. RF, and non-significant results as Not sig.**

| **Anti-PD1** | **Anti-CTLA4** | **Anti-PD1 and Anti-CTLA4** | | | | | | | |
|---|---|---|---|---|---|---|---|---|---|
| **Gene** | **HR** | **p-value** | **Significance** | **HR** | **p-value** | **Significance** | **HR** | **p-value** | **Significance** |
| MSR1 | 0.56 | 0.000026 | Low sig. RF | 0.37 | 0.000048 | Low sig. RF | 0.34 | 0.014 | Low sig. RF |
| CD80 | 0.55 | 0.0001 | Low sig. RF | 0.4 | 0.00032 | Low sig. RF | 0.38 | 0.034 | Low sig. RF |
| **OLR1** | 0.66 | 0.0042 | Low sig. RF | 0.54 | 0.049 | Low sig. RF | 4.33 | 0.04 | High sig. RF |
| **ABCA1** | 0.66 | 0.013 | Low sig. RF | 0.55 | 0.025 | Low sig. RF | 0.18 | 0.013 | Low sig. RF |
| ITGAV | 1.44 | 0.0084 | High sig. RF | 0.72 | 0.21 | Not sig. | 0.29 | 0.047 | Not sig. |
| ADAM 17 | 1.15 | 0.33 | Not sig. | 0.62 | 0.077 | Low sig. RF | 0.2 | 0.0026 | Low sig. RF |
| ANO6 | 0.78 | 0.11 | Not sig. | 0.38 | 0.00018 | Low sig. RF | 0.16 | 0.0002 | Low sig. RF |
| **TMEM245** | 0.7 | 0.022 | Low sig. RF | 0.4 | 0.00026 | Low sig. RF | 0.17 | 0.0095 | Low sig. RF |
| CLEC5A | 1.26 | 0.084 | Not sig. | 0.77 | 0.32 | Not sig. | 0.5 | 0.14 | Not sig. |
| **ATP13A3** | 0.74 | 0.027 | Low sig. RF | 0.49 | 0.0055 | Low sig. RF | 0.48 | 0.25 | Low sig. RF |

Different combinations of these genes, as shown in Table 2, were evaluated.

**Table 2. Results of hazard ratio (HR) of the indicated combination of genes. The significance of the values is indicated as low significant risk factor; low sig. RF, high significant risk factor; high sig. RF, and non-significant results as Not sig.**

| | | **Anti-PD1** | | | **Anti-CTLA4** | | | **Anti-PD1 and Anti-CTLA4** | | |
|---|---|---|---|---|---|---|---|---|---|---|
| **No. of qenes** | **Genetic signature** | **HR** | **p-value** | **Significance** | **HR** | **p-value** | **Significance** | **HR** | **p-value** | **Significance** |
| 10 | *MSR1, CD80, OLR1, ABCA1, ITGAV, ADAM17, ANO6*, *TMEM245, CLEC5A, ATP13A3* | 0.8 | 0.06 | Not sig. | 0.4 | 0.002 | Low sig. RF | 0.2 | 0.0018 | Low sig. RF |
| 7 | *OLR1, ABCA1, ITGAV, ANO6*, *TMEM245, CLEC5A, ATP13A3* | 0.7 | 0.052 | Not sig. | 0.4 | 0.00096 | Low sig. RF | 0.2 | 0.0022 | Low sig. RF |
| 5 | *OLR1, ABCA1, ITGAV, TMEM245, ATP13A3* | 0.7 | 0.06 | Not sig. | 0.4 | 0.002 | Low sig. RF | 0.2 | 0.0021 | Low sig. RF |
| 4 | *OLR1, ABCA1, ITGAV, TMEM245* | 0.7 | 0.069 | Not sig. | 0.4 | 0.0078 | Low sig. RF | 0.2 | 0.0021 | Low sig. RF |
| **4** | ***OLR1, ABCA1, TMEM245, ATP13A3*** | **0.7** | **0.019** | **Low sig. RF** | **0.4** | **0.0079** | **Low sig. RF** | **0.3** | **0.0087** | **Low sig. RF** |
| 3 | *OLR1, ABCA1, TMEM245* | 0.6 | 0.003 | Low sig. RF | 0.5 | 0.017 | Low sig. RF | 0.3 | 0.0087 | Low sig. RF |
| 3 | *OLR1, ABCA1, ATP13A3* | 0.7 | 0.03 | Low sig. RF | 0.5 | 0.0049 | Low sig. RF | 0.2 | 0.007 | Low sig. RF |
| **3** | ***ABCA1, TMEM245, ATP13A3*** | **0.7** | **0.019** | **Low sig. RF** | **0.4** | **0.0079** | **Low sig. RF** | **0.3** | **0.0087** | **Low sig. RF** |
| 3 | *OLR1, TMEM245, ATP13A3* | 0.8 | 0.039 | Low sig. RF | 0.4 | 0.00095 | Low sig. RF | 0.3 | 0.077 | Not sig. |

When these genes were used as a signature using a mean expression of the genes (4 genes in total), an association with favorable result (reduced risk factor or HR (hazard ratio) was observed, being statistically significant (Fig. 1). The combinations with the smaller number of genes, for example 3 genes, also predict a better prognosis in the present analysis in a statistically significant manner.

Furthermore, a genetic signature was evaluated using a mean expression of 7 genes in total, observing an association with the favorable result (reduced risk factor or HR), being statistically limiting in significance for anti-PD1 treatment, and the association was highly significant for anti-CTLA4 therapies or both combined (Fig. 2).

## Claims

1. An *in vitro* method for predicting the response of a subject to an immunotherapy, preferably wherein the immunotherapy comprises at least one immune checkpoint inhibitor, wherein the method comprises the following steps:
a) determining the expression levels of genes *ABCA1, TMEM245,* and *ATP13A3*, in an isolated biological sample from the subject; and
b) comparing the expression levels of the genes determined in step a) with control values, and detecting a significant deviation with respect to those control values, wherein said comparison and detection of the significant deviation allows predicting whether the subject will respond to said immunotherapy.

2. An *in vitro* method for identifying a subject who will respond to an immunotherapy, preferably wherein the immunotherapy comprises, at least, one immune checkpoint inhibitor, wherein the method comprises the following steps:
a) determining the expression levels of genes *ABCA1, TMEM245,* and *ATP13A3*, in an isolated biological sample from the subject; and
b) comparing the expression levels of the genes determined in step a) with control values, and detecting a significant deviation with respect to those control values, wherein detecting said significant deviation allows identifying a subject who will respond to said immunotherapy.

3. The method according to claim 1 or 2, wherein the immunotherapy comprises a regulatory T-cells (Tregs)-targeted therapy.

4. The method according to any one of claims 1 to 3, wherein the immunotherapy comprises, at least, one immune checkpoint inhibitor selected from the list consisting of a PD-1 inhibitor, a PDL-1 inhibitor, a CTLA-4 inhibitor, a TIGIT inhibitor, and any combination thereof.

5. The method according to any one of claims 1 to 4, wherein step a) further comprises determining the expression levels of the *OLR1* gene.

6. The method according to any one of claims 1 to 5, wherein step a) further comprises determining the expression levels of, at least, one gene selected from the list consisting of: *ITGAV, ANO6*, *CLEC5A, MSR1, CD80, AOAM17,* and any combination thereof.

7. The method according to any one of claims 6, wherein the expression levels comprise the expression levels of genes *OLR1, ABCA1, TMEM245, ATP13A3*, *ITGAV, ANO6*, and *CLEC5A.*

8. The method according to any one of claims 1 to 7, wherein the subject has cancer.

9. The method according to any one of claims 1 to 8, wherein the isolated biological sample is from a tumor.

10. The method according to claim 9, wherein the tumor is selected from the list consisting of breast cancer tumor, malignant tumor of the melanocytes, bladder tumor, esophageal adenocarcinoma tumor, glioblastoma tumor, hepatocellular carcinoma tumor, head and neck squamous cell carcinoma tumor, non-small cell lung cancer tumor, small cell lung cancer tumor, gastric tumor, and urothelial tumor.

11. The method according to any one of claims 9 or 10, wherein the tumor comprises elevated levels of Treg lymphocytes.

12. Use of the expression levels of genes *ABCA1, TMEM245,* and *ATP13A3* for the *in vitro* prediction of the response of a subject to an immunotherapy, preferably wherein the immunotherapy comprises, at least, one immune checkpoint inhibitor.

13. *In vitro* use of the expression levels of genes *ABCA1, TMEM245,* and *ATP13A3* for identifying a subject who will respond to immunotherapy, preferably wherein the immunotherapy comprises, at least, one immune checkpoint inhibitor.

14. Use according to claim 12 or 13, further comprising the use of the expression levels of *OLR1* gene.

15. A kit comprising means for the *in vitro* determination of the expression levels of *ABCA1, TMEM245,* and *ATP13A3* genes, in an isolated biological sample from the subject.
